(19) Europäisches Patentamt
European Patent Office
Office européen des brevets

(11) Numéro de publication : **0 008 548**
**B1**

(12) **FASCICULE DE BREVET EUROPÉEN**

(45) Date de publication du fascicule du brevet : **09.06.82**

(51) Int. Cl.³ : **C 07 D 277/78**

(21) Numéro de dépôt : **79400540.5**

(22) Date de dépôt : **31.07.79**

(54) **Nouveau procédé de préparation du disulfure de benzothiazyle.**

(30) Priorité : **18.08.78 FR 7824093**

(43) Date de publication de la demande : **05.03.80 (Bulletin 80/05)**

(45) Mention de la délivrance du brevet : **09.06.82 Bulletin 82/23**

(84) Etats contractants désignés : **BE CH DE FR GB IT LU NL SE**

(56) Documents cités :
**US A 2 024 567**
**US A 2 024 575**

(73) Titulaire : **P C U K PRODUITS CHIMIQUES UGINE KUHLMANN**
**Service Propriété Industrielle Tour Manhattan**
**F-92087 Paris La Défense 2 Cedex 21 (FR)**

(72) Inventeur : **Alicot, Michel Jean Camille**
**La Barthe de Neste**
**F-65300 Lannemezan (FR)**
Inventeur : **Tignol, Adrien Paul Noel**
**15, rue des Pyrénées**
**F-31210 Montrejeau (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

EP 0 008 548 B1

Nouveau procédé de préparation du disulfure de benzothiazyle

La présente invention concerne un nouveau procédé de préparation du disulfure de benzothiazyle :

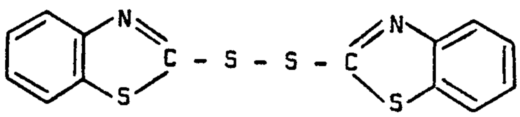

Ce produit est largement utilisé en particulier comme accélérateur de vulcanisation dans l'industrie de la transformation des caoutchoucs naturels et synthétiques ; il peut l'être seul ou en combinaison avec d'autres accélérateurs du type thiazoles, thiurames ou arylguanidines.

D'une manière générale, les procédés de fabrication connus font appel à l'oxydation du mercaptobenzothiazole. Ils se différencient essentiellement les uns des autres par le choix de l'oxydant. Ainsi ont été préconisés : l'acide nitrique (brevet des Etats-Unis d'Amérique n° 1.880.421 - 04.10.1932), le chlorure de nitrosyle et l'oxyde azotique (brevet allemand n° 1.143.203 - 14.08.1963), le nitrite de sodium en milieu acide (brevet des Etats-Unis d'Amérique n° 3.062.825 - 06.11.1962), le chlore (brevet français n° 2.174.052 - 26.02.1973), l'hypochlorite de sodium (brevet anglais n° 559.338 - 15.02.1944), l'eau oxygénée en milieu acide minéral (brevets des Etats-Unis d'Amérique n° 2.024.575 - 17.12.1935 et n° 2.024.567 - 17.12.1935). Certains procédés font également appel à l'oxydation directe du mercaptobenzothiazole par l'oxygène en présence de catalyseurs (par exemple, le brevet des Etats-Unis d'Amérique n° 3.654.297 - 04.04.1972).

Si ces procédés permettent pour la plupart d'obtenir du disulfure de benzothiazyle avec de bons rendements, ils peuvent présenter des inconvénients : l'exploitation n'est pas aussi aisée que peut le laisser supposer la simplicité du schéma réactionnel.

Citons les procédés mettant en jeu un sel alcalin du mercaptobenzothiazole, un sel oxydant, un milieu solvant autre que l'eau, ou qui nécessitent (toujours dans des proportions importantes) l'emploi d'acides minéraux.

Les eaux-mères qui résultent de ces procédés ne sont généralement pas directement recyclables sans préjudice pour la qualité du produit et le rendement, ce qui entraîne nécessairement des traitements complémentaires coûteux. Lorsque le rejet des eaux-mères est envisagé, il faut tenir compte de la forte demande chimique en oxygène des effluents. Les problèmes deviennent d'une complexité telle que ces procédés ne sont plus économiquement viables. Par ailleurs, le disulfure de benzothiazyle obtenu dans nombre de ces procédés contient encore du mercaptobenzothiazole libre, qu'il est nécessaire d'éliminer par un traitement annexe, lui-même générateur de rejet.

Or, il a été trouvé, conformément à la présente invention, qu'il est possible de pallier ces inconvénients. Le procédé selon l'invention, qui consiste à oxyder le mercaptobenzothiazole en suspension aqueuse au moyen de l'eau oxygénée, est caractérisé en ce que l'oxydation est effectuée en présence d'acide éthylène-diaminotétraacétique ou d'un sel alcalin en dérivant, l'acide ou le sel étant utilisé à raison de 0,025 à 1 % en poids par rapport à l'eau oxygénée.

La granulométrie du mercaptobenzothiazole est comprise entre 5 et 200 microns, préférentiellement entre 10 et 100 microns. Une granulométrie plus élevée donnerait lieu à des difficultés d'oxydation.

La concentration de la suspension aqueuse en mercaptobenzothiazole est comprise entre 100 et 300 grammes/litre : des concentrations plus faibles diminuent inutilement la productivité de l'appareillage alors que des concentrations plus fortes peuvent entraîner des difficultés de manutention du mélange réactionnel.

L'eau oxygénée, en excès molaire de 5 à 30 % par rapport au mercaptobenzothiazole, et préférentiellement en excès molaire de 10 à 20 %, est engagée sous la forme de solutions aqueuses, telles que les solutions commerciales usuelles titrant 50 ou 70 % d'eau oxygénée à 100 %.

En vue de la réaction d'oxydation, on utilise l'acide éthylène diaminotétraacétique ou l'un de ses sels alcalins à raison de 0,025 à 1 % en poids, préférentiellement de 0,1 à 0,2 %, par rapport à l'eau oxygénée exprimée en 100 %. Ces proportions sont très inférieures aux proportions d'acide sulfurique préconisé par le brevet US 2 024 567.

Si l'oxydation peut être conduite entre + 10 °C et + 100 °C, on choisit préférentiellement l'intervalle allant de + 20 °C à + 40 °C, dans lequel les réactions secondaires sont tout à fait négligeables. La durée d'oxydation peut être comprise entre 30 minutes et 50 heures : elle est directement liée à la température et également à l'excès d'eau oxygénée. Elle est d'autant plus élevée que la température est plus basse et l'excès d'eau oxygénée plus faible.

Le disulfure de benzothiazyle est formé avec un rendement pratiquement quantitatif ; il est isolé par tout moyen usuel connu tel que filtration, essorage. Il est obtenu dans un état de pureté tel qu'il n'est pas nécessaire d'effectuer un traitement quelconque de purification. En particulier, la teneur en mercaptobenzothiazole libre est inférieure à 0,2 %-0,3 %. Le disulfure est formé de particules élémentaires dont la taille va de 1 à 2 microns, réunies en agglomérats de 30 à 50 microns ; cet aspect physique est tout à fait satisfaisant pour les applications dans les caoutchoucs naturels et synthétiques et permet en particulier une dispersion parfaite.

Les exemples suivants illustrent l'invention sans la limiter.

Exemple 1

Dans un réacteur en acier inoxydable préalablement passivé, de 100 litres de capacité, muni d'un dispositif d'agitation, d'une prise de température et d'une double enveloppe permettant de régler la température, on introduit sous agitation 56 litres d'eau, 11,5 grammes d'un agent dispersant du type condensat d'oxyde d'éthylène avec un acide gras, 1,7 gramme du sel disodique de l'acide éthylènediaminotétraacétique et 14,820 kg de mercaptobenzothiazole à 100 %. On chauffe à la température de 35 °C et ajoute, en quinze minutes environ, 1,770 kg d'eau oxygénée à 100 % sous forme de solution à 70 %. La température est maintenue à 35 °C. On laisse sous agitation pendant 24 heures. On filtre la suspension obtenue ; le filtrat est lavé avec environ 5 fois 6 litres d'eau, essoré et séché à la température de 80 à 100 °C.

On obtient 14,580 kg de disulfure de benzothiazyle se présentant sous la forme d'une poudre de couleur crème, de point de fusion (non corrigé) de 172-174 °C, dont la teneur en mercaptobenzothiazole libre est de 0,15 %.

Le rendement par rapport au mercaptobenzothiazole est de 99 %.

Exemple 2

On procède comme indiqué à l'exemple 1, mais en utilisant au lieu de 56 litres d'eau pure, 56 litres d'eaux-mères provenant d'une précédente opération ; ce volume correspond à la totalité des eaux-mères auxquelles a été ajoutée la plus grande partie des eaux de lavage.

On obtient, suivant le même mode opératoire, 14,500 kg de disulfure de benzothiazyle, de point de fusion (non corrigé) 172-174 °C, de teneur en mercaptobenzothiazole libre égale à 0,12 %. L'aspect physique, en particulier la couleur du produit, est identique à celui du produit obtenu à l'exemple 1.

Le rendement en disulfure de benzothiazyle est de 98,5 % par rapport au mercaptobenzothiazole.

Le mode opératoire, décrit dans chacun de ces exemples, peut être aménagé sans difficulté en vue d'opérations en continu.

**Revendications**

1. Procédé pour la préparation du disulfure de benzothiazyle par oxydation du mercaptobenzothiazole en suspension aqueuse au moyen de l'eau oxygénée, caractérisé en ce que l'oxydation est effectuée en présence d'acide éthylènediaminotétraacétique ou d'un sel alcalin en dérivant, l'acide ou le sel étant utilisé à raison de 0,025 à 1 % en poids par rapport à l'eau oxygénée.

2. Procédé tel que revendiqué selon la revendication 1 dans lequel le mercaptobenzothiazole a une granulométrie comprise entre 5 et 200 microns.

3. Procédé tel que revendiqué sous chacune des revendications 1 et 2 dans lequel la concentration en mercaptobenzothiazole est comprise entre 100 et 300 g/l.

4. Procédé tel que revendiqué sous 1 dans lequel l'eau oxygénée est présente en excès, celui-ci étant un excès molaire de 5 à 30 % par rapport au mercaptobenzothiazole, de préférence 10 à 20 %.

5. Procédé tel que revendiqué selon chacune des revendications 1 et 4 dans lequel l'acide ou le sel est utilisé à raison de 0,1 à 0,2 % en poids par rapport à l'eau oxygénée.

6. Procédé tel que revendiqué sous 1 caractérisé en ce qu'il est effectué en continu.

**Claims**

1. Process for the preparation of benzothiazyl disulphide by the oxidation of mercaptobenzothiazole in an aqueous suspension by means of hydrogen peroxide, characterised in that the oxidation takes place in the presence of ethylenediaminotetra-acetic acid or an alkali metal salt derivative, the acid or salt being used at the rate of 0.025 to 1 % by weight in relation to the hydrogen peroxide.

2. Process as claimed in claim 1, in which mercaptobenzothiazole has a granulometry of between 5 and 200 microns.

3. Process as claimed in each of claims 1 and 2, in which the mercaptobenzothiazole concentration is between 100 and 300 g/l.

4. Process as claimed in claim 1, in which the hydrogen peroxide is present in excess, the latter being a molar excess of 5-30 % in relation to the mercaptobenzothiazole, but preferably 10 to 20 %.

5. Procedure as claimed in each of claims 1 and 4, in which the acid or the salt is used at a rate of 0.1 to 0.2 % in relation to the hydrogen peroxide.

6. Process as claimed in claim 1, characterised in that it is carried out continuously.

**Ansprüche**

1. Verfahren zur Herstellung von Benzothiazyldisulfid durch Oxidation von Mercaptobenzothiazol in wäßriger Suspension mit Wasserstoffperoxid, dadurch gekennzeichnet, daß man die Oxidation in Gegenwart von Äthylendiamintetraessigsäure oder eines davon abgeleiteten Alkalimetallsalzes durchführt, wobei die Säure oder das Salz in einer Menge von 0,025 bis 1 Gew.-%, bezogen auf das Wasserstoffperoxid, verwendet wird.

2. Verfahren nach Anspruch 1, dadurch gekennzeichnet, daß man Mercaptobenzothiazol mit einer Korngröße zwischen 5 und 200 µm einsetzt.

3. Verfahren nach einem der Ansprüche 1 und 2, dadurch gekennzeichnet, daß man eine Mercaptobenzothiazol-Konzentration zwischen

100 und 300 g/l anwendet.

4. Verfahren nach Anspruch 1, dadurch gekennzeichnet, daß man Wasserstoffperoxid in einem molaren Überschuß von 5 bis 30 %, vorzugsweise von 10 bis 20 %, bezogen auf Mercaptobenzothiazol, anwendet.

5. Verfahren nach den Ansprüchen 1 und 4, dadurch gekennzeichnet, daß man die Säure oder das Salz in einer Menge von 0,1 bis 0,2 %, bezogen auf Wasserstoffperoxid, verwendet.

6. Verfahren nach Anspruch 1, dadurch gekennzeichnet, daß man es kontinuierlich führt.